# EUROPÄISCHE PATENTSCHRIFT

(11) **EP 2 299 901 B1**
(45) Veröffentlichungstag und Bekanntmachung des Hinweises auf die Patenterteilung: **07.08.2013**
(21) Anmeldenummer: 09757127.7
(22) Anmeldetag: 03.06.2009
(51) Int. Cl.: A61B 5/00, A61B 5/097

(54) **TRANSPORTABLER PNEUMOTACHOGRAPH ZUR MESSUNG VON BESTANDTEILEN DES EXSPIRATIONSVOLUMENS**
PORTABLE PNEUMOTACHOGRAPH FOR MEASURING COMPONENTS OF AN EXPIRATION VOLUME
PNEUMOTACHOGRAPHE TRANSPORTABLE DESTINÉ À LA MESURE DE COMPOSANTES D'UN VOLUME D'AIR EXPIRÉ

(30) Priorität: 05.06.2008 DE 102008027630; 05.06.2008 DE 202008007748 U
(43) Veröffentlichungstag der Anmeldung: 30.03.2011
(73) Patentinhaber: Aerocrine AB, 169 67 Solna (SE)
(72) Erfinder: EICHLER, Rüdiger, 97225 Zellingen (DE)
(74) Vertreter: Holmberg, Martin Tor
(86) Internationale Anmeldenummer: PCT/DE2009/000791
(87) Internationale Veröffentlichungsnummer: WO 2009/146693

(56) Entgegenhaltungen:
- WO-A-2007/120898
- US-A1- 2005 065 446
- US-A1- 2005 083 527
- US-A1- 2005 240 115
- US-A1- 2006 201 503
- US-A1- 2007 169 776
- US-A1- 2007 221 224

## Beschreibung

Die Erfindung betrifft einen transportablen Pneumotachographen zur Messung von Bestandteilen, insbesondere von NO, des Exspirationsvolumens.

Stickoxide und andere gasförmige Verbindungen in der Ausatemluft werden zur Beurteilung des physischen Zustandes des Menschen herangezogen, denn sie sind Indikatoren für Stoffwechselprozesse im Organismus, Störungen und Erkrankungen des Menschen. Stationäre Geräte zur diagnostischen Gasanalyse der Ausatemluft sind bekannt und seit langer Zeit auf dem Markt erhältlich.

Ein transportabler Gasanalysator mit einem NO-Sensor ist in der EP 1 439 781 beschrieben, bei dem der Patient bei einer vorgeschriebenen Strömungsgeschwindigkeit und einem vorgeschriebenen Druck ausatmet. Nachteilig an dem dort beschriebenen Gerät ist, dass keine spirometrischen Messdaten erfasst werden können, die eine Korrelation von erfasstem Messwert zu dem entsprechenden erkrankten Lungenbereich ermöglichen. Eine Konditionierung des Mess- bzw. Kalibriergases vor dem Sensor erfolgt mittels eines Nafion® - Schlauchs, mit welchem die Feuchte des Mess- bzw. Kalibriergases auf Werte der Umgebungsluft gesetzt werden.

In der EP 0 973 444 B1 ist eine Vorrichtung und ein Verfahren zur Bestimmung des NO-Gehaltes in der Ausatemluft mit einer Erstvorrichtung, um den Stickoxidanteil im zeitlichen Verlauf während der Exhalationsphase zu bestimmen, offenbart, wobei während der Anfangsphase die Exhalation gegen keinen oder nur einen geringen Gegendruck und danach gegen einen Widerstand oder Gegendruck erfolgt.

Auch sind Geräte zur Lungenfunktionsanalyse, der Spirometrie seit Jahren in der Praxis in der Verwendung.

Die Spirometrie ist ein Verfahren zur Lungen-Funktionsprüfung. Dabei werden Lungen- und Atemvolumina gemessen und graphisch im Spirogramm dargestellt. Zur Erfassung der Lungenvolumina wird ein Spirometer oder auch Pneumotachograph benötigt.

Der Patient atmet über ein Mundstück in ein Atemrohr, wobei die Nase mit einer Nasenklemme verschlossen wird. Dabei misst das Spirometer elektronisch über einen Flusssensor die Luftströmungsgeschwindigkeit, mit der ein- und ausgeatmet wird, und daraus wird die Menge der geatmeten Luft pro Zeit berechnet. Die Luftmengen, die bei diesen Atemzügen bewegt werden, bildet das Gerät grafisch ab. So kann auch ein direkter Vergleich der Messwerte aus verschiedenen Tests erfolgen.

Durch Messung der Luftströmungsgeschwindigkeit oder Ausatemgeschwindigkeiten und der Lungenvolumina ist es dem Arzt möglich, Erkrankungen der Lunge zu diagnostizieren und in ihrem Verlauf zu kontrollieren. Folgende Werte können mit Hilfe der Spirometrie gemessen werden:
Atemzugvolumen (AZV): Es entspricht dem ein- bzw. ausgeatmeten Volumen bei normalem Atemzug.
Inspiratorisches Reservevolumen (IRV): Dies ist das Volumen, das nach normaler Einatmung noch zusätzlich eingeatmet werden kann.
Expiratorisches Reservevolumen (ERV): Es ist das Volumen, das nach normaler Ausatmung noch zusätzlich ausgeatmet werden kann.
Inspiratorische Kapazität (IC): Sie ist definiert als das Volumen, das nach normalem Ausatmen maximal eingeatmet werden kann.

Vitalkapazität (VC) ist das Volumen, das nach maximaler Einatmung maximal ausgeatmet werden kann.

Einsekundenkapazität (FEV1, Tiffeneau-Test) ist das Volumen, das bei maximaler Einatmung in einer Sekunde maximal ausgeatmet werden kann.

Diese Messgrößen helfen beispielsweise zwischen den beiden Hauptgruppen von Lungenerkrankungen zu unterscheiden:
Obstruktive Lungenerkrankungen: Sie werden durch eine Verengung der Atemwege verursacht, z.B. durch Asthma oder COPD.
Restriktive Lungenerkrankungen: Dabei sind Lunge und/oder Brustkorb vermindert dehnbar. Beispiele sind Lungenverhärtung (Lungenfibrose), Flüssigkeitsansammlung im Lungenspalt (Pleuraerguss) oder ein hoch stehendes Zwerchfell (Zwerchfellparese).

Bei der Spirometrie atmet der Patient über ein Mundstück ein bzw. aus. Das Mundstück ist mit einem Spirometer verbunden und in den meisten Fällen mit einem Bakterienfilter versehen. Zur Erfassung der verschiedenen Messgrößen muss der Patient die Anweisungen des Untersuchenden bezüglich Ein- und Ausatmung genau befolgen. Sonst werden falsche Werte gemessen, die wiederum zu falschen Rückschlüssen bei der Behandlung führen können. Die Untersuchung hängt somit von einer guten Mitarbeit des Patienten ab.

Ein weiteres Gerät zur Messung von NO im Ausatemvolumen ist in der US-Schrift 6 010 459 beschrieben. Hier erfolgt eine Spirometrie nach der Messwerterfassung. Der Patient atmet dort synthetische mit NO versetzte und nachträglich befeuchtete Luft ein. Bei Ausatmen muss der Patient einen definierten Druck im Messgerät erzeugen, vorgeblich, damit sich das Gaumen-Rachensegel beim Ausatmen schließt und keine, die Messung verfälschende Luft des Nasen-Rachenraums in den Ausatemstrom gerät, in welchem die NO-Konzentration das bis zu 100 fache im Vergleich zur Lungenausatemluft betragen kann. Nachteilig an diesem Gerät ist die Ortsgebundenheit, da zur Messung das synthetische Gas zum Einatmen verwendet werden muss.

Ein transportabler Pneumotachograph zur Messung von Bestandteilen des Exspirationsvolumens ist aus der deutschen Schrift DE 20 2007 003 818.6 bekannt. Hier erfolgt eine Messung von Gasbestandteilen ohne vorherige Konditionierung des Mess- oder des Kalibriergases.

Zudem liegt mit den 2005 veröffentlichten Guidelines der ATS/ERS (Exhaled breath condensate: methodological recommendations and unresolved questions. I. Horvath, J. Hunt and P.J. Barnes, On behalf of the ATS/ERS Task Force on Exhaled Breath Condensate, Eur Respir J 2005; 26: 523 - 548) erstmals eine Gesamtdarstellung der Methoden zur Atemkondensatdiagnostik vor. Guidelines zur NO-Messung sind in den Schriften "ATS Workshop Proceedings: Exhaled Nitric Oxide and Nitric Oxide Oxidative Metabolism in Exhaled Breath Condensate: Executive Summary. Am J Respir Crit Care Med. 2006 Apr 1;173(7):811-813" und "American Thoracic Society Documents: ATS Workshop Proceedings: Exhaled Nitric Oxide and Nitric Oxide Oxidative Metabolism in Exhaled Breath Condensate, Proc Am Thorac Soc Vol 3. pp 131-145, 2006" dargelegt.

US 2007/0221224 A1 zeigt ein System für die Lieferung von einem Fluss von Atemgas zu einem Atemweg eines Patienten. Das System beinhaltet ein unter Druck stehendes Durchflussmodul, welches einen unter Druck stehenden Fluss von Atemgas erzeugt und einen an dem Fluss angkoppelten Patientenkreiswelcher Kreis den Fluss von Atemgas eines Atemweges kommuniziert.

US 2007/0169776 A1 beschreibt ein Druckunterstützungssystem, dass eine Patientschaltung, ein Anschlussmodul und ein Tank umfasst. Die Patientenschaltung liefert einen unter Druck stehenden Fluss von Atemgas zu einem Patienten.

US 2005/0065446 A1 beschreibt eine Methode zur Erfassung und Nachweis von Verbindungen in einer menschlichen Atemprobe.

US 2005/0240115 A1 erläutert ein System und Verfahren der Konditionierung einer Probe und der Umgebung eines Ausatemluft-Sensors. Es umfasst (i) die Verwendung eines speziellen Molekularsiebs für einen dreifachen Zweck: (a) Beibehaltung des Sensors bei einer konstanten relativen Luftfeuchtigkeit während der Lagerung, (b) Äquilibrierung des einkommenden Atemstromes auf die gleiche relative Luftfeuchtigkeit des Sensors, und (c) Beseitigung des Analyten aus der Umgebung des Sensors während der Lagerung.

US 2005/0083527 A1 zeigt bloß ein Einweg-Sensor für den Einsatz mit einem Gerät, dass in einer gasförmigen Probe Analyten quantifiziert. Der Sensor besteht aus (i) ein Sensorelement, (ii) ein Mittel für die Anbindung des Einweg-Sensors mit einem GasAnalyse-Gerät, und (iii) ein Gehäuse.

Es ist zu konstatieren, dass die Standardisierung der Probennahme, Probenlagerung und Analytik noch zu verbessern ist. Die Standardisierung der Probennahme muß zukünftig ebenfalls erfolgen. Speziell bei der Analytik ist daher den verwendeten Verfahren und deren Validierung mehr Aufmerksamkeit zu schenken.

Gaskonditionierungsvorrichtungen sind ebenfalls im Stand der Technik bekannt. In der einfachsten Form werden beispielsweise Gase durch Einsprühen von feinnebeligem Wasser befeuchtet. Alternativ werden Gase befeuchtet, indem sie durch oder entlang Wasser getränkter Materialien wie feuchte Tücher, feuchte Filter etc geführt werden.

Eine Entfeuchtung von Gasen wird durch Silikagele oder andere Wasser entziehende Verbindungen erreicht. Darüber hinaus können Gase mittels Trocknungs- und Befeuchtungsschläuchen ent- oder befeuchtet werden. Ein solcher Schlauch wird von der Fa. Permapure bereitgestellt und ist im Handel unter der Markenbezeichnung Nafion® erhältlich.

Für die kontinuierliche Trocknung von Gasen wird das Nafion® als Rohr extruiert. Ein Nafion®-Rohr oder ein Bündel von Nafion®-Rohren ist von einem Gehäuse umgeben und wird mit einem Trockengas im Gegenstrom beströmt. Dadurch wird zwischen Gas und Trockengas ein Wasserdampf-Partialdruckgefälle aufrecht gehalten und dem Gas kontinuierlich Feuchte entzogen.

Eine Befeuchtung von Gasen erfolgt mit einem analogen System. Hier wird das Nafion®-Rohr im Gegenstrom mit Wasser beströmt. Eine Temperierung des Gases kann mittels eines Thermostats im Wasserkreislauf erfolgen. Nachteilig an dieser Vorrichtung ist, dass es zu einer Übersättigung und damit zu einer Kondensation von Wasser im Gas führenden Nafion®-Schlauch kommen kann. Das im Gas mitgeschleppte Wasser führt dann zu Störungen des Messsensors.

Mit den genannten Systemen werden zuverlässig somit nur Werte von 0% oder 100% relativer Feuchte erzielt. Nachteilig an denen aus dem Stand der Technik bekannten Be- und Entfeuchtungssystemen ist, dass eine Konditionierung von Gasen auf Werte beispielsweise von 50%, 60% oder 70% relativer Feuchte mit diesen Systemen gegebenenfalls nur annähernd erreicht werden kann.

Der Erfindung liegt daher das technische Problem zu Grunde, einen Pneumotachographen zum Messen von NO im Ausatemvolumen anzugeben, der transportabel und handlich ist und eine Korrelation von erfassten Messdaten eines oder mehrerer Bestandteile des Ausatemstromes mit einer Lungenfunktionsprüfung erlaubt, wobei die Analytik unter Beachtung standardisierender Guidelines erfolgen kann und eine Lokalisierung des Krankheitsherdes ermöglicht.

Insbesondere besteht bei der Verwendung von elektrochemischen Sensoren das Problem von extremer Sensitivität dieser Sensoren gegenüber Temperatur und Feuchtegehalt der Gase. Zudem weisen diese Sensoren Querempfindlichkeiten gegenüber weiteren möglichen Bestandteilen des Ausatemgases auf. Bei elektrochemischen Sensoren zur Bestimmung von NO findet sich hier oft eine Querempfindlichkeit gegenüber NO₂. Insofern der Einfluß der genannten Parameter unberücksichtigt bei der Messwerterfassung bleibt, werden nicht vergleichbare Messwerte ermittelt, die zwangsläufig zu falschen Diagnosen führen müssen.

Insbesondere wird bei dieser Sensortechnologie eine exakte Gaskonditionierung benötigt. Messsensoren zur Bestimmung von Gasbestandteilen werden beispielsweise im Bereich der Rauchgasanalyse oder in der Medizintechnik bei der Atemgasanalyse eingesetzt. Insbesondere werden in der Gasanalytik elektrochemische Sensoren eingesetzt, welche jedoch eine hohe Empfindlichkeit gegenüber der Gasfeuchte und der Gastemperatur aufweisen.

Sensortypen, bei welchen eine Konditionierung des Mess- oder Kalibriergases in vorgeschlagener Weise zu genaueren Messdaten führt, sind in erster Linie elektrochemische Gassensoren, insbesondere NO-, CO-, CO₂- oder O₂-Sensoren.

Gelöst wird das technische Problem, indem die Erfindung einen transportablen Pneumotachographen gemäβ Anspruch 1 zur Bestimmung von Bestandteilen im Exspirationsvolumen mit einem Prozessor, einem ausatemseitig am Pneumotachographen angebrachtes PEEP-Ventil, einen einatemseitig am Pneumotachographen angebrachten Filter zum Entfernen des Anteils an zu bestimmendem Bestandteil in der Inspirationsluft angibt. Im oder am Pneumotachographenrohr ist mindestens eine Probennahme angebracht und diese mit einem Ventil verbunden. Außerhalb des Pneumotachographenrohres ist mindestens ein Sensor zur Bestimmung eines der Bestandteile im Expirationsvolumen eingerichtet. Downstreamseitig zum Sensor befindet sich eine Pumpe zur Förderung des Probe- oder Kalibriergases. Zwischen dem Ventil und einem Sensor ist ein chemisch und/oder physikalisch modifizierter Schlauch, welcher von einer Klimatisierungskammer zur Konditionierung des den Schlauch durchströmenden Probengases und/oder Kalibrierungsgases umschlossen ist, eingerichtet. Die zu konditionierenden Proben- und/oder Kalibriergase sind im Schlauch durch die Klimatisierungskammer leitbar. In der Klimatisierungskammer ist eine definierte Gasatmosphäre einstellbar, so dass mittels der Klimatisierungskammer die relative Feuchte und/oder die Temperatur und/oder die Zusammensetzung des den Schlauch durchströmenden Gases auf vorgegebene Parameter einstellbar ist bzw. sind.

Der Sensor kann aus der Gruppe "elektrochemischer Sensor, Chemilumineszenssensor, enzymatischer Sensor, Immunoassay-Sensor, NO-Sensor, O₂-Sensor, H₂O₂-Sensor, CO₂-Sensor, CO-Sensor, Sensor für Biomarker, Ion mobility spectrometry (IMS)-Sensor" und/oder eine Kombinationssensorik aus den genannten Sensoren ausgewählt sein. Je nach zu analysierendem Bestandteil (NO, O₂) der Ausatemluft ist der entsprechende Sensor einsetzbar. Es können auch mehrere Sensoren zum gleichzeitigen Messen verschiedener Bestandteile eingerichtet sein.

Der Begriff "definierte Gasatmosphäre" beschreibt zusammengefasst die chemischen und physikalischen Zustände in der Klimatisierungskammer. Die Klimatisierungskammer ist gasgefüllt. Das Gas weist eine definierte Feuchte, Temperatur und Zusammensetzung auf. Vorzugsweise ist dies ein Gas, welches frei von den zu bestimmenden Bestandteilen des Exspirationsvolumens ist. Die in der Klimatisierungskammer eingestellte Temperatur und Feuchte sind vorzugsweise Werte, welche denen des optimalen Arbeitsbereiches des verwendeten Sensors entsprechen und in welchen die Messwerteerfassung nur in geringen Schwankungsbreiten erfolgt, womit eine stabile und reproduzierbare Messwerteerfassung gewährleistet ist.

Der Begriff "probe- und/oder kalibriergasführende Leitung" bezeichnet die von der Probennahme bzw. Einlass des Kalibriergases zum Konditionierungsschlauch führenden Gasleitungen sowie die vom Austritt aus der Klimatisierungskammer bis zur Messkammer führenden Gasleitungen. Der Bereich der Messkammer sowie die zum Auslass führende Gasleitung ist ebenfalls von diesem Begriff umfasst.

Der Feuchtegehalt sowie die Temperatur des Exhalates bei Austritt aus der Lunge sind Schwankungen unterworfen und als nicht konstant zu betrachten. Eine weitere nicht kontrollierbare Veränderung dieser Werte erfolgt bei Eintritt ins Pneumotachographenrohr. Die Ursachen hierfür sind unterschiedlicher Natur. Einerseits kann beispielsweise das Exhalat abgekühlt werden, wenn es im Pneumotachographenrohr kühler als in der Lunge ist, beispielsweise aufgrund einer gegenüber der Lunge deutlich geringeren Umgebungstemperatur und dementsprechend niedrigeren Temperatur des Inspirationsstroms. Dies bedingt in diesem Fall, da die relative Feuchte ein von der Temperatur abhängiger Wert ist, dass die relative Feuchte im Pneumotachographenrohr ansteigen würde. Allerdings kann eine Veränderung der genannten Werte auch durch eine Mischung mit Restgasen unterschiedlichsten Ursprungs (Restexhalat des vorherigen Atemzuges, Kalibriergasreste, Reste des Inhaltes etc.) bedingt sein. Hierbei könnte aufgrund eines "Verdünnungseffektes" aufgrund einer Mischung mit trockneren Gasen eine Reduzierung der relativen Feuchte eintreten. Ebenfalls können Temperaturschwankungen auftreten.

Aus diesen Fallbeispielen wird deutlich, dass die zu messenden Gasproben nicht vorhersagbare Parameter bezüglich Temperatur und Feuchte aufweisen, worauf dann aufgrund der Empfindlichkeit des Sensors gegenüber diesen Parametern es zu nicht vergleichbaren Messwerten kommt.

Mit der Klimatisierungskammer wird erreicht, dass Feuchte und Temperatur der aus dem Exspirationsstrom entnommenen Gasprobe auf die Werte in der Klimatisierungskammer eingestellt werden. Die den Sensor erreichende Gasprobe weist dann nahezu konstante Werte bezüglich Temperatur und Feuchte auf. Die in diesem Temperatur- und Feuchtefenster beispielsweise ermittelten NO-Konzentrationen verschiedener Gasproben sind folglich vergleichbar, da die die Messung beeinflussenden Parameter konstant gehalten werden.

Das Prinzip der Konditionierung erfolgt auf Basis des Ausgleichs von Konzentrations- und Temperaturgefällen. Beim Durchströmen des Schlauches gleichen sich die Differenzen der genannten Parameter zwischen der Klimatisierungskammer und dem Schlauchvolumen aus, wodurch sich Feuchtegehalt und Temperatur angleichen.

Mittels der Klimatisierungskammer kann die relative Feuchte des den Schlauch durchströmenden Gases auf Werte von 0% bis 100% relative Feuchte, entsprechend dem Wert in der Klimatisierungskammer, eingestellt werden. Gleichzeitig erfolgt eine Temperierung des Gases auf die in der Klimatisierungskammer eingestellte Temperatur.

Der Schlauch zur Konditionierung des durchströmenden Probengases und/oder Kalibrierungsgases, im Weiteren Konditionierungsschlauch genannt, weist chemische und/oder physikalische Eigenschaften auf, die einen Ausgleich von Feuchte und Temperatur zulassen. Vorzugsweise ist der in seinen Eigenschaften chemisch und/oder physikalisch modifizierte Schlauch Wasserdampf durchlässig. Die Membraneigenschaften des Schlauchmaterials weisen daher permeable oder semipermeable Eigenschaften betreffend Austausch von Feuchtigkeit und gegebenenfalls auch gegenüber Störverbindungen auf bei gleichzeitig geringen wärmeisolierenden Eigenschaften. Insbesondere handelt es sich um einen Nafion®-Schlauch der Fa. PermaPure.

Das Volumen der Klimatisierungskammer ist vorzugweise um ein Mehrfaches größer als das Schlauchvolumen, um den Einfluß von Feuchte und Temperatur des Probengases oder Kalibriergases auf die Werte in der Klimatisierungskammer möglichst gering zu halten und einen Angleich der Parameter des Proben- oder Kalibriergases auf die Werte in der Klimatisierungskammer zu bewirken. Das Verhältnis zwischen Klimatisierungskammervolumen zu Schlauchvolumen beträgt vorzugsweise 200/1 bis 4/1, vorzugsweise 5/1.

Die Klimatisierungskammer ist vorzugsweise ein zur Umgebung abgeschlossener Raum, durch welchen der Konditionierungsschlauch hindurchgeführt ist. Hierzu sind mindestens zwei Anschlüsse bzw. zwei Durchführungen in der Wand der Klimatisierungskammer als Ein- und Auslass eingerichtet. Die Anschlussverbindungen bzw. Durchführungen sind gegenüber dem Innenraum der Klimatisierungskammer abgedichtet, um einen Austausch der Gase zwischen Innenraum und Schlauchvolumen bzw. der Umgebung zu verhindern.

In der Klimatisierungskammer kann ein Feuchtigkeitssensor oder Temperatursensor oder beides eingerichtet sein. Über diese Sensoren wird der Zustand in der Klimatisierungskammer kontrolliert und im Falle von Abweichungen von den Sollwerten die Abweichung signalisiert. Die Auswertung der Soll/Istwerte erfolgt bevorzugt prozessgesteuert. Das Signal kann ein optisches oder akustisches Signal sein. Zudem kann der Messvorgang bei Eintreten einer Abweichung unterbrochen werden. Auch kann bei Abweichung der Ist-Werte von den Soll-Werten eine prozessgesteuerte Korrektur der Zustandsparameter in der Klimatisierungskammer erfolgen. Hierzu sind zusätzlich Mittel eingerichtet, mit denen eine korrektive Einstellung der Ist-Werte durchgeführt werden kann.

Zusätzlich kann die probe- und/oder kalibriergasführende Leitung vor und/oder hinter der Klimatisierungskammer mit einem oder mehreren Temperatur- und/oder Feuchtigkeitssensoren ausgestattet sein, mit welchen der Ist-Zustand des Proben- und/oder Kalibriergases hinsichtlich dessen Temperatur und/oder Feuchtigkeitsgehaltes vor und/oder nach der Klimatisierungskammer bestimmt werden kann.

Sensoren, welche hinter der Klimatisierungskammer in der probeführenden Leitung eingerichtet sind, dienen u.a. der Kontrolle der tatsächlichen Parameter des Proben- bzw. Kalibiergases nach der Klimatisierung und ggfs. der Feststellung einer Abweichung von dem durch die Klimatisierungskammer vorgegeben Sollwert. Insofern eine Abweichung festgestellt wird, kann anhand der Ist-Werte eine Korrektur der Gasatmosphäre in der Klimatisierungskammer erfolgen und eine Gasatmosphäre eingestellt werden, mit welcher die gewünschten Sollwerte erzielt werden. Eine Korrektur der Temperatur und/oder der Feuchtigkeit des Proben- bzw. Kalibriergases kann aber auch durch eine Anpassung des Flows, welcher die Verweil- und Kontaktzeit des Proben- bzw. Kalibriergases mit der Gasatmosphäre in der Klimatisierungskammer bestimmt, erfolgen. Vorzugsweise erfolgt die Korrektur der Gasatmosphäre und/oder des Flows ebenfalls prozessgesteuert über entsprechende durch den oder die Temperatur- oder Feuchtigkeitssensoren generierte Steuersignale.

Auch können die Ist-Daten von Temperatur und Feuchtegehalt des Proben- bzw. Kalibriergases herangezogen werden, um auf Basis einer mathematischen Korrelation von Ist- und Sollwerten eine Korrektur der Messwerte durchzuführen.

Die Klimatisierungskammer kann mit einem Mittel zum Einstellen einer definierten relativen Feuchte in der Klimatisierungskammer ausgestattet sein. Im einfachsten Fall ist in der Klimatisierungskammer ein wasserspeicherndes Medium eingesetzt. Dies kann ein wassergetränktes Wattepad oder ein wasserspeicherndes Gel sein, mit welchem durch Verdunstung des Wassers eine Sättigung des Gases in der Klimatisierungskammer auf 100% relative Feuchte erzielt wird. Auch ist der umgekehrte Fall von 0% relativer Feuchte erreichbar, indem in die Klimatisierungskammer ein wasserbindendes Medium, vorzugsweise aus Kieselgel eingesetzt wird.

In einer besonderen Ausführungsform ist das Mittel zum Einstellen der Feuchte eine Vorrichtung aufweisend zwei Behälter jeweils enthaltend ein Gas mit einer definierten relativen Feuchte, wobei der Feuchtegehalt der beiden Gase voneinander unterschiedlich ist. Vorzugsweise enthält der eine Behälter ein Gas mit 0% relativer Feuchte und der andere Behälter ein Gas mit 100% relativer Feuchte. Die Behälter können temperierbar ausgestaltet sein. Die Behälter sind über ein gemeinsames regel- und steuerbares Mischventil mit der Klimatisierungskammer verbunden und mittels des Mischventils kann die relative Feuchte des Gases in der Klimatisierungskammer durch Mischung der beiden Gase aus den Behältern auf Basis von in Steuersignale umgewandelter Messwerte des Feuchtigkeitssensors prozessgesteuert eingestellt werden. Vorzugweise ist hinter dem Mischventil zusätzlich eine Mischkammer mit entsprechender Sensorik zur Bestimmung von Temperatur und Feuchte des Mischgases eingerichtet. Erst bei Erreichen der Soll-Werte in der Mischkammer wird das Mischgas an die Klimatisierungskammer weitergeleitet.

In einer bevorzugten Ausführungsform ist die Klimatisierungskammer mit einem Mittel zur Temperierung der Klimatisierungskammer ausgestattet. Vorzugsweise ist dieses mittels des Prozessors regel- und steuerbar. Ein solches Mittel ist insbesondere ein Peltier-Element, mit welchem sowohl geheizt als auch gekühlt werden kann.

Beispielsweise kann auch ein Vorratsreservoir mit konditioniertem Kammergas eingerichtet sein, welches mit der Klimatisierungskammer verbunden ist. Auf Anzeige oder Detektion einer Abweichung der Ist-Werte von den Soll-Werten erfolgt dann ein Austausch der Gasatmosphäre.

Der Volumenstrom des Gases im Schlauch beträgt 1 bis 20 ml/s, vorzugsweise 2 bis 3 ml/s.

Die Maße des Schlauches, wie Länge, Innendurchmesser, Wandstärke sowie der Volumenstrom sind derart gewählt, dass das den Schlauch durchströmende Gas bei Eintritt in die Messkammer des Sensors Feuchte und Temperatur entsprechend der Gasatmosphäre in der Klimatisierungskammer aufweist.

Das im oder am Pneumotachographenrohr angebrachte Ventil ist vorzugsweise ein statisches Dreiwegeventil. Mittels des Dreiwegeventils kann zwischen Kalibrier- und Probengas hin und her geschaltet werden, ohne dass am Pneumotachographen bauliche Veränderungen vorgenommen werden müssen. Das Kalibriergas wird in der Klimatisierungskammer ebenfalls auf sensoroptimierte Werte eingestellt.

In einer bevorzugten Ausführungsform ist in der das Kalibriergas führenden Leitung vor dem Ventil ein zusätzlicher Filter zur Entfernung von die Kalibrierung störenden Bestandteilen aus dem Kalibriergas eingerichtet, welcher derart dimensioniert ist, dass er in Größe und Filtereffektivität für die Kalibriergasmenge und die Strömungsbedingungen am Sensor hergerichtet ist. Vorzugsweise handelt es sich hierbei um einen Aktivkohlefilter. Der Aktivkohlefilter kann in einer Ausführungsform Kaliumpermanganat enthalten.

Der elektrochemische Stickstoffmonoxydsensor weist einen Messbereich von 0 bis 5000 ppb, vorzugsweise 0 bis 3000 ppb, bevorzugt 0 bis 1000 ppb, insbesondere von 0 bis 500 ppb auf.

Der Konditionierungsschlauch kann ganz oder teilweise mit einem Filtermaterial gefüllt und/oder die Innenwand des Schlauches mit einem chemisch oder physikalisch aktiven Film belegt sein, welches die Messung negativ beeinflussende Störverbindungen, insbesondere NO₂ aus dem Kalibrier- oder Probengas entfernt. Das Entfernen kann durch chemische Umsetzung, Adsorption oder Lösen der Verbindung im Film erreicht werden.

Kernstück des transportablen Pneumotachographen zur Bestimmung von Bestandteilen des Exspirationsvolumens ist ein Pneumotachograph an dem vorzugsweise ein austauschbares Mundstück und/oder ein Bakterienfilter angesetzt werden. Durch den Pneumotachographen atmet der Patient ein und aus. Einatemseitig ist am Pneumotachographen ein Filter zum Herausfiltern des zu messenden Bestandteils aus der Umgebungsluft vorgesetzt. Im Pneumotachographen ist in Strömungsrichtung der Ausatmung hinter denen im Pneumotachographenrohr zur Erzeugung eines Strömungswiderstandes befindlichen Lamellen oder Gitter im Rohr, beispielsweise in der Strömungsmitte, oder in der Rohrwand eine Probennnahme eingerichtet.

Die Probennahme ist mit dem Ventil über eine Zuführungsleitung verbunden. Der Sensor ist außerhalb des Pneumotachographenrohres eingerichtet und mit dem Ventil über eine weitere Leitung, die teilweise oder ganz aus dem Konditionierungsschlauch bestehen kann, verbunden. Es können auch mehrere, voneinander unabhängig steuer- und regelbare Probennahmen eingerichtet sein.

In einer besonderen Ausführungsform ist der Pneumotachograph mit mehreren Messeinheiten, wobei eine Messeinheit aus einer Probennahme, einem Ventil, einer Zuführungsleitung zum Sensor sowie dem Sensor selbst besteht, ausgestattet. Neben Sensoren mit Gaskonditionierung können somit auch Sensoren ohne Gaskonditionierung im Pneumotachographen eingerichtet sein. Auch kann je nach Sensortyp zwischen einer Zuführung der Gase mit oder ohne Konditionierung des Gases durch eine prozessgesteuerte Schaltung entschieden werden, wobei zwischen einer Zuleitung zum Sensor ohne Klimatisierung und einer Zuleitung zum Sensor mit Klimatisierung hin- und her geschaltet werden kann, ohne dass für jeden Sensor eine eigene Klimatisierungskammer eingerichtet werden muß.

Sensortypen, bei welchen eine Konditionierung des Proben- oder Kalibriergases in vorgeschlagener Weise zu genaueren Messdaten führt, sind insbesondere NO-, CO-, CO₂- oder O₂-Sensoren. Diese Aufzählung ist nicht abschließend.

Das Ventil kann nach der Maßgabe des das Pneumotachographenrohr passierenden Inspirations- oder Exspirations- oder eines Teilinspirations- oder Teilexspirationsvolumens oder -stroms den Zustand "offen" oder "geschlossen" haben, wobei das Ventil durch den Prozessor ansteuerbar ist. Hiermit wird erreicht, dass eine Probennahme bzw. Messung nur dann durchgeführt wird, wenn definierte Volumenströme oder Teilvolumenströme der Inspiration oder der Exspiration das Pneumotachographenrohr passieren. Der Zeitpunkt, wann eine Messung zu erfolgen hat, wird über die vom Pneumotachographen ermittelte Fluß-Volumen-Korrelation berechnet, welcher mit einem Prozessor verbunden ist, an welchem auch das Ventil angeschlossen und von diesem ansteuerbar ist. Da die Probennahme prozessorgesteuert direkt aus dem Pneumotachographen erfolgt, wird jede Zeitverzögerung oder Diskordanz zwischen Flowmessung und Probennahme primär ausgeschlossen bzw. werden kompensatorische Korrekturen vorgenommen.

Die Ausatmung kann gegen einen exspiratorischen Widerstand, der durch das ausatemseitig am Pneumotachographenrohr angebrachte PEEP-Ventil erzeugt wird, erfolgen. Der exspiratorische Widerstand beträgt vorzugsweise 5 bis 20 cm H₂O und bedingt, dass sich der Atemwegsmitteldruck und die funktionelle Residualkapazität erhöhen.

Mittels des PEEP-Ventils wird erreicht, dass die Entlüftung der Lunge bzw. Lungenbläschen auch bei einer Atemwegsverengung, Sekret in den Atemwegen oder andersartigen Belüftungsstörungen (Verteilungsstörung) gleichmäßiger erfolgt. Diese Maßnahme ist Voraussetzung für eine reproduzierbare Wiederholung der Atemmanöver und einer weitgehend ungestörten Emission der Bestandteile der Ausatemluft während des Messvorganges.

In einer besonderen Ausführungsform ist das PEEP-Ventil abnehmbar eingerichtet. Hierdurch kann der transportable Pneumotachograph auch zur Messung von spirometrischen Daten verwendet werden, ohne dass ein weiteres Spirometer bereitgestellt werden muß. Für die Zeit der Aufnahme der spirometrischen Messdaten ist der Messsensor vorzugsweise außer Betrieb gesetzt oder abgeschaltet.

In einer besonderen Ausführungsform ist das PEEP-Ventil ein Doppelventil, welches den Flow zwischen einem Mindestflow und einem Maximalflow begrenzt. Anders ausgedrückt: Das Ventil öffnet bei Erreichen eines zum Mindestflow in Korrelation stehenden ersten Ausatemdruck, wodurch die Strömungsgeschwindigkeit im Pneumotachographenrohr sprunghaft vom Wert "Null" auf die Strömungsgeschwindigkeit des Mindestflow ansteigt. Der Patient kann nun bei einem definierten Flow ausatmen. Bei Überschreiten eines zweiten gegenüber dem ersten Ausatemdruck grö-βeren zweiten Ausatemdruck, der mit dem Maximalflow korreliert, schließt das Ventil wieder und der Wert der Strömungsgeschwindigkeit fällt sprunghaft auf den Wert "Null". Der Patient ist nun an der Ausatmung gehindert. Ein Ausatmen ist nur zwischen dem Mindestflow und dem Maximalflow möglich. Ein solches PEEP-Dopelventil weist beispielsweise eingangsseitig (pneumotachographenseitig) ein federbelastetes Rückschlagventil und ausgangsseitig (Umgebung) ein Druckventil auf, wobei das federbelastete Rückschlagventil erst bei Erreichen bzw. Überschreiten eines eingangsseitig erzeugten ersten Druckes öffnet und das Druckventil ausgangsseitig bei Überschreiten eines eingangsseitig erzeugten höheren Staudruckes, der größer als der erste Druck (Öffnungsdruck) ist, schließt. In anderen Worten ausgedrückt: Das PEEP-Ventil öffnet an seiner Eingangsöffnung erst ab Überwindung eines ersten definierten Druckswiderstandes. Ab diesen Zeitpunkt ist dessen Ausgangsöffnung geöffnet. Bei Steigerung des Flows erhöht sich gleichzeitig der Druck auf das Rückschlagventil und die Rückschlagsfeder wird weiter zusammengedrückt und drückt gleichzeitig in Richtung der Ausgangsöffnung. Rückseitig zwischen Verschluß des Rückschlagsventils und der Feder ist beispielsweise ein Kegel angebracht, der beim Zusammendrücken der Feder in die Ausgangsöffnung einfasst und diese sukzessive verschließt. Mit dieser Art PEEP-Ventil kann der exspiratorische Flow und der exspiratorische Widerstand zwischen einem Mindestwert und einem Maximalwert, die jeweils einstellbar sind, begrenzt werden.

Zur Kontrolle des Flows weist der transportable Pneumotachograph eine optische oder akustische Kontrolle auf, anhand derer der Patient seine Ausatmung kontrollieren und einstellen kann. Die optische Kontrolle des Exspirationsstroms kann aus der Gruppe "y-t Graph, Balkengraphik, Leuchtdiodenanzeige mit einer oder mehreren Leuchtdioden" ausgewählt sein. Die akustische Kontrolle kann ein Piepton oder ein sich in der Lautstärke oder Frequenz ändernder Ton sein.

In einer bevorzugten Ausführungsform ist zwischen dem einatemseitig am Pneumotachographen angebrachten Filter, der austauschbar eingerichtet ist, und dem Pneumotachographenrohr ein Ventil oder eine Klappe eingerichtet, welches bei der Exspiration die Eintrittsöffnung des Pneumotachographen verschließt. Hiermit wird erreicht, dass der Patient nur gegen den durch das PEEP-Ventil bewirkten exspiratorischen Widerstand atmet. Vorzugsweise schließt das Ventil oder die Klappe selbsttätig. Ein solches Ventil kann ein Rückschlagventil, ein Sperrventil oder eine einfache Klappe, die sich in das Pneumotachographenrohr öffnet, sein. Aber es sind hier keine Begrenzungen bauteiletechnischer Art vorgesehen.

Der transportable Pneumotachograph kann einen oder mehrere gasundurchlässige Sammelbehälter zum Auffangen von Proben und/oder von Atemvolumina mehrerer Atemzüge aufweisen. Mit diesen können mehrere Atemzüge vereinigt oder Proben bis zur Analyse aufbewahrt werden.

Die Oberflächen der probeführenden Leitungen oder der Luftführungskanäle des erfindungsgemäßen Pneumotachographen können modifiziert und dergestalt sein, dass auf diesen Membranen, flüssige Filme aufgetragen oder Einlage von porösen Schichten oder Membranen eingearbeitet sind, so dass bestimmte Bestandteile der Probengase zurückgehalten bzw. in den Schichten chemisch gebunden werden. Die Aufzählung ist beispielhaft und nicht begrenzend. So können die Analyse störende Substanzen bzw. Chemikalien wie beispielsweise am Sensor Kreuzempfindlichkeiten auslösende Verbindungen wie NO₂ aus dem Exspirationsstrom oder Inspirationsstrom physikalisch oder chemisch gebunden werden, um eine störungsfreie Analyse gewährleisten zu können. Andererseits können freie Radikale durch chemische Umsetzung von in oder an den modifizierten Oberflächen deaktiviert und so die Probe stabilisiert werden.

Der transportable Pneumotachograph kann zusätzlich mit einer Spüleinrichtung ausgestattet sein, die es erlaubt, dass der Sensor und /oder die Probenahmeeinheit (Probennahme, Ventil, zuführende Leitungen, Schlauch) mit einem Gas ausgewählt aus der Gruppe "Bestandteilsfreie Luft, synthetische Luft, zum Zwecke der Kalibrierung hergerichteten Gase" oder einer Kombination der genannten Gase gespült und damit von der Ausatemluft gereinigt wird, um die Genauigkeit der Messungen zu erhöhen. Hierzu kann eine Pumpe eingerichtet sein, die beispielsweise mit dem einatemseitig am Pneumotachographen angebrachten Filter über eine, vorzugsweise separate Leitung verbunden ist und Umgebungsluft durch den Filter in den Probennahme- und Sensorbereich oder in einen von beiden Bereichen pumpt. Es können zudem ein oder mehrere Anschlüsse zum Anschließen einer unter Druck stehenden oder druckfreien Spülgasflasche eingerichtet sein.

In einer weiteren Ausführungsform kann eine Kalibrierung des Sensors mit einem Gas, ausgewählt aus der Gruppe "Bestandteilsfreie Luft, synthetische, zum Zwecke der Kalibrierung hergerichteten Gase" oder einer Kombination der genannten Gase nach einer oder einer Anzahl von Messeinheiten durchgeführt werden. Die Kalibrierwerte der genannten Gase sind individuell am Gerät einstellbar.

Die bestandteilsfreie Luft kann erzeugt werden, indem Raumluft einatemseitig durch den am Pneumotachographen angebrachten Filter gepumpt wird. Diese gereinigte Luft wird anschließend in der Klimatisierungskammer konditioniert und anschließend über den Sensor geleitet.

Um die Gefahr einer Fehlmessung durch Beimischung von Nasenluft zu vermindern können folgende Maßnahmen vorgesehen sein:
● Tragen einer Nasenklammer bei der Inspiration
● Ausatmung mit konstantem Flow bzw. Flow grösser Null, denn ein Stopp des Flow während des Ausatemmanövers bringt Luft aus der Nase in den Rachen.

Ein konstanter Flow ist ein Flow mit einer maximalen Abweichung von +/- 10% vom Mittelwert. Der konstante Flow der exspiratorischen Luft kann vorzugsweise 10 - 500 ml/s, insbesondere 45 bis 55 ml/s betragen. Vorzugsweise beträgt dieser 50 ml/s. Er kann aber auch variiert werden.

Der exspiratorische Flow muss für eine Dauer von 1 bis 30 s, vorzugsweise von 2 bis 10 s, insbesondere von 4 bis 6 s, konstant gehalten werden.

Der Flow von 50 ml/s sollte in einem Bereich von +/- 10 % für 4 s bei Kindern unter 12 Jahren oder 6 s bei Kindern über 12 und Erwachsenen gehalten werden. Das entspricht bei einem Flow von 50 ml/s etwa 300 ml Luft insgesamt.

Die Anpassung an die für die Analyse eines oder mehrerer Bestandteil der Ausatemluft benötigten Flowraten und Drücke beispielsweise aufgrund geforderter Rahmenbedingungen durch gesetzliche Bestimmungen oder Richtlinien erfolgt durch Auswahl und Einsatz eines den geforderten Flow und Druck maßgebendes PEEP-Ventil oder bei einem regelbaren PEEP-Ventil durch Einstellung dessen an die geforderten Parameter.

Während dieses Plateau bleibt der NO-Messwert in einem Bereich entsprechend den Richtlinien der American Thoracic Society (ATS) und der Europäischen Respiratorischen Gesellschaft (ERS), welche im Dezember 2004 von der ATS und im Juni 2004 von der ERS verabschiedet worden sind, erhalten.

Die Messung ist zu wiederholen. Eine Messung unterliegt den ATS/ERS-Richtlinien, wenn mindestens zwei Atemmanöver den Kriterien entsprechen.

In einer weiteren Ausführungsform kann ein Teil- oder das Gesamtexspirationsvolumen in einem oder mehreren gasundurchlässigen Auffangbehältnis, vorzugsweise einem Gasbeutel, aufgefangen werden. Alternativ kann auch das Volumen des Pneumotachographen diese Aufgabe übernehmen.

In einer bevorzugten Ausführungsform weist das Pneumotachographenrohr im Einlass für die inspiratorische Luft ein Ventil oder eine Klappe, die verschließbar ist, und mehrere Auslässe für die exspiratorische Luft auf, wobei in den Auslässen für die exspiratorische Luft Auslassventile eingerichtet sind. Mittels des Durchflussmessers wird der Volumenstrom der exspiratorischen Luft ermittelt und auf Basis dieses Wertes dieser mittels einer prozessorgestützten Berechnung in zuerst theoretische Werte von Teilvolumenströme zerlegt. Jedem dieser Teilvolumenströme kann eine Zone des Respirationstraktes zugewiesen werden. Das Ventil oder die Klappe im Einlass für die inspiratorische Luft und/oder die Auslassventile sind durch den Prozessor steuer- und regelbar, wobei der Betriebszustand "zu" (a) oder "auf" (b) des Ventils oder der Klappe im Einlass und/oder der Auslassventile entsprechend einem bestimmten Teilvolumenstrom des Volumenstroms der exspiratorischen Luft einstellbar ist.

Damit kann anhand des Wertes des Exspirationsvolumens eine Zuordnung zu zeitlich nacheinander ausgeatmeten Teilvolumenströme eines Exspirationsvolumens erfolgen, wobei diesen Teilvolumenströme bestimmten Regionen und Zonen des Respirationstraktes zugewiesen werden können. Durch die Operation können Erkrankungen und Störungen des Respirationstraktes lokalisiert werden.

Eine weitere Ausführungsform sieht vor, dass eine Messung von Bestandteilen des Exspirationsvolumens nur dann erfolgt, wenn ein definiertes Teilexspirationsvolumen das Pneumotachographenrohr, insbesondere die Probennahme passiert. Durch die spirometrische Messdatenerfassung kann über den Prozess berechnet werden, zu welchem Zeitpunkt ein Teilvolumenstrom x, der aus einem Bereich Y der Lunge stammt, in dem beispielsweise ein Krankheitsherd vermutet wird, insbesondere die Probennahme passiert. Eine Messung der Bestandteile nur dieses Teilvolumenstroms kann dann gestartet werden.

Unter den Bedingungen von Standards, beispielsweise der ATS/ERS-Richtlinien, sind nur Messwerte auswertbar und repräsentativ, die unter definierten Messbedingungen gewonnen wurden. Hierzu ist vorgesehen, dass eine Messung von Bestandteilen des Exspirationsvolumens nur bei Eintreten des oder der vorgegebenen Parameter des Exspirationsvolumens "Überwinden des exspiratorischen Widerstands" und/oder "konstanter exspiratorischen Flow" und/oder "Dauer des exspiratorischen Flows" erfolgt. Werden diese Parameter nicht erreicht oder nicht lange genug gehalten, wird kein Messwert aufgenommen, d.h. dass Sensorsignal wird vom Prozessor nicht ausgewertet. Insofern keine Signalübernahme erfolgt oder ein Bedingung nach Einleitung der sensorischen Messung nicht eingehalten wurde, kann ein Messwert, der unter Nicht-Standard-Bedingungen ermittelt wurde, als ein solcher gekennzeichnet und ausgegeben werden.

Vorzugsweise können die Werte der Parameter "Überwinden des exspiratorischen Widerstands" und/oder "konstanter exspiratorischen Flow" und/oder "Dauer des exspiratorischen Flows" individuell eingestellt und/oder diese in Abhängigkeit von der Patientengruppe und/oder dem Zustand des Exspirationstraktes des Patienten aus einem Speichermedium, vorzugsweise dem Prozessor, abgerufen werden.

Nachstehend wird die Erfindung anhand zweier Ausführungsbeispiele näher erläutert:
Fig.1: schematische Darstellung des transportablen Pneumotachographen
Fig.2: schematische Darstellung einer alternativen Ausführungsform

In den Figuren stellen durchgezogene Linie Leitungen zur Führung des Proben- und/oder Kalibriergases, Punkt-Strichlinien stellen elektronische Verbindungen zur Daten oder Signalübertragung dar.

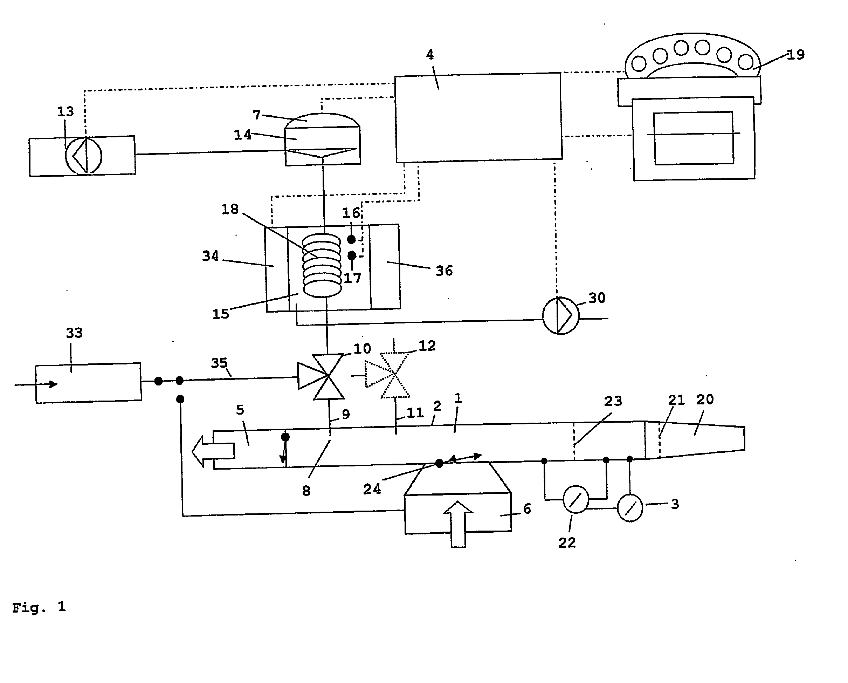

Die Figur 1 zeigt einen transportablen Pneumotachographen zur Bestimmung von Bestandteilen des Exspirationsvolumen mit einem Pneumotachographen 1 mit einem Pneumotachographenrohr 2, einem Mittel zur Druckmessung 3 und einem Prozessor 4, einem ausatemseitig am Pneumotachographen 1 angebrachtes PEEP-Ventil 5, einem einatemseitig am Pneumotachographen 1 angebrachten Filter 6 zum Entfernen des Anteils an zu bestimmendem Bestandteil in der Inspirationsluft und einem Sensor 7. Im Kanal des Pneumoptachographenrohres 2 ist eine Probennahme 8 mit einer Leitung 9, welche durch die Wand des Pneumotachorgraphenrohres 2 geführt ist, eingerichtet. Die Probennahme 8 ist über die Leitung 9 mit dem Ventil 10, hier ein Dreiwegeventil, verbunden.

Zusätzlich ist eine zweite Probennahme 11 mit ebenfalls einem Dreiwegeventil 12 eingerichtet, welche (nicht dargestellt aus Gründen der Übersichtlichkeit) mit einem zweiten Sensor verbunden ist. Die Zuführungsleitung zum Sensor kann ebenfalls mit einer Klimatisierungskammer ausgestattet sein. Insofern eine solche aufgrund des gewählten Sensortyps nicht notwendig ist, ist diese entbehrlich und das Gas wird ohne Konditionierung in die Messkammer geleitet.

Downstreamseitig ist am Sensor 7 eine Pumpe 13 zur Förderung des Kalibrier- oder Probengases eingerichtet. Zwischen Ventil 10 und der Messkammer 14 des Sensors 7 befindet sich die Klimatisierungskammer 15, durch welche der Konditionierungsschlauch 18 geführt ist. In der Klimatisierungskammer 15 ist ein wassergetränktes Wattepad 36 eingelegt, mit welchem die Gasatmosphäre in der Klimatisierungskammer 15 mit Feuchtigkeit gesättigt wird. Die Klimatisierungskammer 15 ist mit einem Temperatursensor 16 und einem Feuchtigkeitssensor 17 ausgestattet, die mit dem Prozessor 4 verbunden sind. Die Klimatisierungskammer 15 hat zum Temperieren der Klimatisierungskammer 15 ein Peltier-Element 34. Zur Temperierung kann alternativ zum Peltier-Element ein Widerstandsheizelement in Form eines Heizfolienelements verwendet werden.

Das Pneumotachographenrohr 2 weist eine optische Kontrolle 19 des Exspirationsstroms auf. Das Mundstück 20 ist mit einem Bakterienfilter 21 ausgestattet. Der Pneumotachograph 1 weist standardgemäß ein elektrisches Manometer 22 auf, welches den Druckunterschied vor und hinter den Lamellen 23 misst.

Zwischen dem einatemseitig am Pneumotachographen 1 angebrachten Filter 6 und dem Pneumotachographenrohr 2 ist eine inspiratorisch wirksame Klappe 24 eingerichtet, welche bei der Exspiration die Eintrittsöffnung des Pneumotachographen 1 verschließt.

Durchgezogene Linie stellen Leitungen, Punkt-Strichlinien stellen elektronische Verbindungen zur Daten oder Signalübertragung dar.

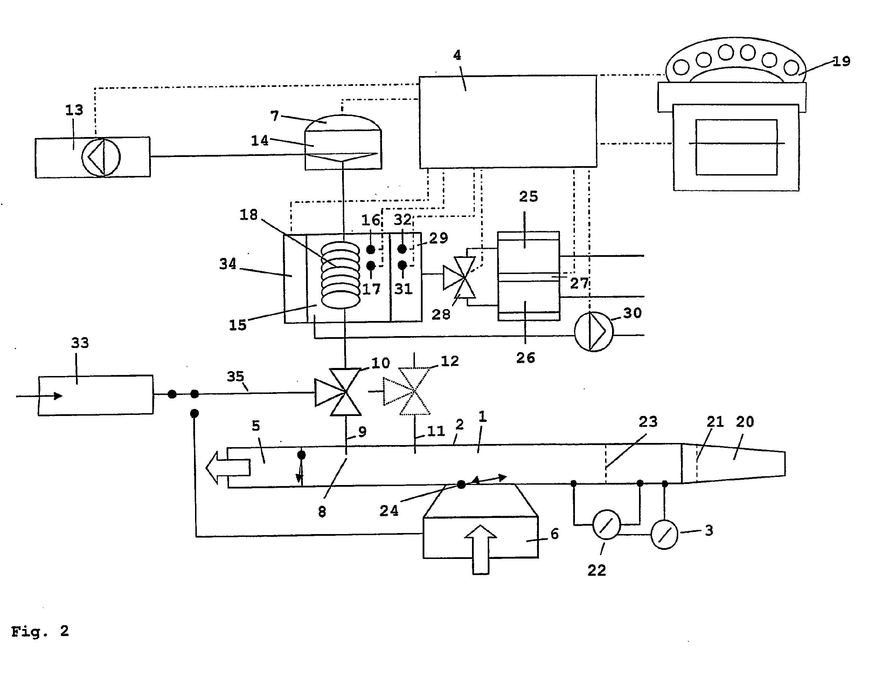

Die Figur 2 zeigt eine alternative Ausführungsform des transportablen Pneumotachographen 1. Die Klimatisierungskammer 15 ist mit einem Mittel 15 zum Einstellen einer definierten relativen Feuchte in der Klimatisierungskammer ausgestattet. Diese Vorrichtung besteht aus zwei Behältern 25, 26 jeweils enthaltend ein Gas mit einer definierten relativen Feuchte, wobei der Feuchtegehalt der beiden Gase voneinander unterschiedlich ist. Der eine Behälter 25 enthält ein Gas mit 0% relativer Feuchte und der andere Behälter 26 ein Gas mit 100% relativer Feuchte. Die Behälter 25, 26 sind mittels eines Heizelementes 27 temperierbar. Die Behälter 25, 26 sind über ein gemeinsames über den Prozessor 4 regel- und steuerbares Mischventil 28 mit der Klimatisierungskammer 15 verbunden. Mittels des Mischventils 28 kann die relative Feuchte des Gases in der Klimatisierungskammer 15 durch Mischung der beiden Gase aus den Behältern 25, 26 auf Basis von in Steuersignale umgewandelter Messwerte des Feuchtigkeitssensors 17 prozessgesteuert eingestellt werden.

Hinter dem Mischventil 28 kann eine Mischkammer 29 mit einem Temperatur- 32 und Feuchtigkeitssensor 31 zur Bestimmung von Temperatur und Feuchte des Mischgases eingerichtet sein. Alternativ können die Mischkammer 29 und die Klimatisierungskammer 15 eine Einheit bilden.

Das Mischgas wird mittels der Mischpumpe 30 in die Klimatisierungskammer 15 befördert. Die Klimatisierungskammer 15 ist ebenfalls mit einem Temperatur- 16 und einem Feuchtigkeitssensor 17 ausgestattet, die mit dem Prozessor 4 verbunden sind. Ebenso sind das Mischventil 28 sowie die dazugehörige Mischpumpe 30 mit dem Prozessor 4 verbunden. Die Klimatisierungskammer 15 weist zudem ein Peltier-Element 34 zum Temperieren der Klimatisierungskammer auf.

In dieser Ausführungsform ist in der das Kalibriergas führenden Leitung 35 vor dem Ventil 10 ein zusätzlicher Filter 33 zur Entfernung von die Kalibrierung störenden Bestandteilen aus dem Kalibriergas eingerichtet, welcher derart dimensioniert ist, dass er in Größe und Filtereffektivität für die Kalibriergasmenge und die Strömungsbedingungen am Sensor hergerichtet ist.

### Bezugszeichenliste

- 1: Pneumotachographen
- 2: Pneumotachographenrohr
- 3: einem Mittel zur Druckmessung
- 4: einem Prozessor
- 5: PEEP-Ventil
- 6: Filter
- 7: Sensor
- 8: Probennahme
- 9: Leitung
- 10: Ventil
- 11: zweite Probennahme
- 12: zweites Ventil
- 13: Pumpe
- 14: Messkammer des Sensors
- 15: Klimatisierungskammer
- 16: Temperatursensor Klimatisierungskammer
- 17: Feuchtigkeitssensor Klimatisierungskammer
- 18: Konditionierungsschlauch
- 19: optische Kontrolle
- 20: Mundstück
- 21: Bakterienfilter
- 22: Manometer
- 23: Lamellen, Blende oder Sieb
- 24: inspiratorisch wirksame Klappe
- 25: Behälter
- 26: Behälter
- 27: Temperierungselement
- 28: Mischventil
- 29: Mischkammer
- 30: Mischpumpe
- 31: Feuchtigkeitssensor
- 32: Temperatursensor
- 33: Kalibriergasfilter
- 34: Temperierungselement
- 35: Kalibiriergasleitung
- 36: Wattepad

## Patentansprüche

1. Transportabler Pneumotachograph zur Bestimmung von Stickstoffmonoxyd (NO) in einem Messbereich von 0 bis 500 ppb im Exspirationsvolumen mit einen Prozessor (4), ein ausatemseitig am Pneumotachographen (1) angebrachtes PEEP-Ventil (5), einen einatemseitig am Pneumotachographen (1) angebrachten Filter (6) zum Entfernen des Anteils an NO in der Inspirationsluft, mindestens einer im oder am Pneumotachographenrohr (2) angebrachten Probennahme (8), einem mit der Probennahme (8) verbundenen Ventil (10), mindestens einem außerhalb des Pneumotachographenrohres eingerichteten Sensor (7) zur Bestimmung von NO im Expirationsvolumen und einer downstreamseitig zum Sensor (7) angeordneten Pumpe (13), **dadurch gekennzeichnet, dass** zwischen dem Ventil (10) und dem Sensor (7) ein chemisch und/oder physikalisch modifizierter Schlauch (18), welcher von einer Klimatisierungskammer (15) zur Konditionierung des den Schlauch (18) durchströmenden Probengases und/oder Kalibrierungsgases umschlossen ist, eingerichtet ist, wobei die zu konditionierenden Proben und/oder Kalibriergase im Schlauch durch die Klimatisierungskammer leitbar sind, und in der Klimatisierungskammer (15) eine definierte Gasatmosphäre einstellbar ist, so dass mittels der Klimatisierungskammer (15) die relative Feuchte und/oder die Temperatur und/oder die Zusammensetzung des den Schlauch (18) durchströmenden Proben- und/oder Kalibriergases auf vorgegebene Parameter einstellbar ist.

2. Transportabler Pneumotachograph nach Anspruch 1, **dadurch gekennzeichnet, dass** die relative Feuchte des den Schlauch (18) durchströmenden Gases auf Werte von 0% bis 100% relative Feuchte einstellbar ist.

3. Transportabler Pneumotachograph nach Anspruch 1 oder 2, **dadurch gekennzeichnet, dass** der chemisch und/oder physikalisch modifizierter Schlauch (18) Wasserdampf durchlassig ist und vorzugsweise ein Nafion®-Schlauch ist.

4. Transportabler Pneumotachograph nach einem der Ansprüche 1 bis 3, **dadurch gekennzeichnet, dass** in der Klimatisierungskammer (15) ein Feuchtigkeitssensor (17) und/oder Temperatursensor (16) eingerichtet ist.

5. Transportabler Pneumotachograph nach einem der Ansprüche 1 bis 4, **dadurch gekennzeichnet, dass** der Volumenstrom des Gases im Schlauch (18) 1 bis 20 ml/s, vorzugsweise 2 bis 3 ml/s betragt und abhängig von der Spülzeit der Messkammer (14) des Sensors (7) und des Zuleitungssystems (8, 9, 10, 18, 33, 35) zum Sensor (7) einstellbar ist.

6. Transportabler Pneumotachograph nach einem der Ansprüche 1 bis 5, **dadurch gekennzeichnet, dass** die Klimatisierungskammer (15) mit einem, vorzugsweise regel- und steuerbaren, Mittel zur Temperierung der Klimatisierungskammer (34) ausgestattet ist.

7. Transportabler Pneumotachograph nach Anspruch 6, **dadurch gekennzeichnet, dass** das Mittel zur Temperierung (34) ein Peltier-Element oder ein Widerstandsheizelement ist.

8. Transportabler Pneumotachograph nach einem der Ansprüche 1 bis 7, **dadurch gekennzeichnet, dass** die Klimatisierungskammer (15) mit einem Mittel zum Einstellen einer definierten relativen Feuchte in der Klimatisierungskammer (15) ausgestattet ist.

9. Transportabler Pneumotachograph nach Anspruch 8, **dadurch gekennzeichnet, dass** das Mittel zum Einstellen der Feuchte ein wasserspeicherndes Medium, vorzugsweise ein wassergetränktes Wattepad, ist oder ein wasserbindendes Medium, vorzugsweise aus Kieselgel ist.

10. Transportabler Pneumotachograph nach Anspruch 8, **dadurch gekennzeichnet, dass** das Mittel zum Einstellen der Feuchte eine Vorrichtung aufweisend zwei Behälter (25, 26) jeweils enthaltend ein Gas mit einer definierten relativen Feuchte, wobei der Feuchtegehalt der beiden Gase voneinander unterschiedlich ist, wobei die Behälter (25, 26) über ein gemeinsames regel- und steuerbares Mischventil (28) mit der Klimatisierungskammer (15) verbunden sind, ist und mittels des Mischventils (28) die relative Feuchte des Gases in der Klimatisierungskammer (15) durch Mischung der beiden Gase aus den Behältern (25, 26) auf Basis von in Steuersignale umgewandelter Messwerte des Feuchtigkeitssensors (17) prozessgesteuert einstellbar ist.

11. Transportabler Pneumotachograph nach Anspruch 10, **dadurch gekennzeichnet, dass** zur Temperierung der Behälter (25, 26) ein Heizelement (27) eingerichtet ist.

12. Transportabler Pneumotachograph nach Anspruch 10 oder 11, **dadurch gekennzeichnet, dass** zwischen Klimatisierungskammer (15) und Mischventil (28) eine Mischkammer (29) mit einem Feuchtigkeitssensor (31) und einem Temperatursensor (32) eingerichtet ist.

13. Transportabler Pneumotachograph nach einem der Ansprüche 1 bis 12, **dadurch gekennzeichnet, dass** das Ventil (10) ein statisches Dreiwegeventil ist.

14. Transportabler Pneumotachograph nach einem der Anspruche 1 bis 13, **dadurch gekennzeichnet, dass** das Pneumotachographenrohr (2) im Einlass für inspiratorische Luft ein Ventil oder eine Klappe (24) aufweist, die verschließbar ist, wobei das Pneumotachographenrohr mehrere Auslasse für die exspiratorische Luft aufweist, wobei in den Auslassen für die exspiratorische Luft Auslassventile eingerichtet sind, wobei mittels eines Durchflussmessers der Volumenstrom der exspiratorischen Luft messbar und auf Basis dieses Wertes dieser mittels einer prozessorgestutzten Berechnung in theoretische Werte von Teilvolumenströme zerlegbar ist, wobei die Teilvolumenströme unterschiedlichen Zonen des Respirationstraktes zuweisbar sind, wobei das Ventil oder die Klappe (24) im Einlass für die inspiratorische Luft und/oder die Auslassventile durch den Prozessor (4) ansteuerbar und regelbar sind, wobei der Betriebszustand "zu" (a) oder "auf" (b) des Ventils oder Klappe (24) im Einlass für die inspiratorische Luft und/oder die Auslassventile entsprechend einem bestimmten Teilvolumenstrom des Volumenstroms der exspiratorischen Luft einstellbar ist.

15. Transportabler Pneumotachograph nach einem der Ansprüche 1 bis 14, **dadurch gekennzeichnet, dass** das PEEP-Ventil (5) ein Doppelventil ist, welches den Flow zwischen einem Mindestflow und einem Maximalflow begrenzt.

16. Transportabler Pneumotachograph nach einem der Ansprüche 1 bis 15, **dadurch gekennzeichnet, dass** der chemisch und/oder physikalisch modifizierter Schlauch (18) zur Entfernung von die sensorische Messung störenden Verbindungen im Probe- oder Kalibriergas ganz oder teilweise mit einem Filtermaterial gefüllt und/oder die Innenwand des Schlauches mit einem chemisch oder physikalisch aktiven Film belegt ist.

17. Transportabler Pneumotachograph nach einem der Ansprüche 1 bis 16, **dadurch gekennzeichnet, dass** ein Prozessor (14) zur Steuerung und Regelung der Klimatisierungskammer (15) und/oder der Mischkammer (29) eingerichtet ist.

18. Transportabler Pneumotachograph nach einem der Ansprüche 1 bis 17, **dadurch gekennzeichnet, dass** in der probe- und/oder kalibriergasführende Leitung vor und/oder hinter der Klimatisierungskammer ein oder mehrere Temperatur- und/oder Feuchtigkeitssensoren eingerichtet sind.

## Claims

1. A transportable pneumotachograph for determining nitrogen monoxide (NO) in a measuring range from 0 to 500 ppb in the expiration volume, having a processor (4), a PEEP valve (5) attached to the pneumotachograph (1) on the exhalation side, a filter (6) attached to the pneumotachograph (1) on the inhalation side for removing the portion of NO in the inspiration air, at least one sampling unit (8) attached in or to the pneumotachograph tube (2), a valve (10) connected to the sampling unit (8), at least one sensor (7) provided outside of the pneumotachograph tube for determining NO in the expiration volume, and a pump (13) arranged downstream of the sensor (7), **characterized in that** a chemically and/or physically modified tube (18), which is enclosed by a climate control chamber (15) for conditioning the sample gas and/or calibration gas flowing through the tube (18), is provided between the valve (10) and the sensor (7), wherein the sample and/or calibration gases to be conditioned can be channelled in the tube through the climate control chamber, and a defined gas atmosphere can be set in the climate control chamber (15) such that the relative humidity and/or the temperature and/or the composition of the sample and/or calibration gas flowing through the tube (18) can be set to predefined parameters by means of the climate control chamber (15).

2. The transportable pneumotachograph as claimed in claim 1, **characterized in that** the relative humidity of the gas flowing through the tube (18) can be set to values from 0% to 100% relative humidity.

3. The transportable pneumotachograph as claimed in claim 1 or 2, **characterized in that** the chemically and/or physically modified tube (18) is permeable to water vapor and is preferably a Nafion® tube.

4. The transportable pneumotachograph as claimed in one of claims 1 to 3, **characterized in that** a moisture sensor (17) and/or temperature sensor (16) is provided in the climate control chamber (15).

5. The transportable pneumotachograph as claimed in one of claims 1 to 4, **characterized in that** the volume flow of the gas in the tube (18) is 1 to 20 ml/s, preferably 2 to 3 ml/s, and can be adjusted depending on the purging time of the measuring chamber (14) of the sensor (7) and of the feed pipe system (8, 9, 10, 18, 33, 35) to the sensor (7).

6. The transportable pneumotachograph as claimed in one of claims 1 to 5, **characterized in that** the climate control chamber (15) is equipped with a, preferably regulatable and controllable, means for controlling the temperature of the climate control chamber (34).

7. The transportable pneumotachograph as claimed in claim 6, **characterized in that** the means for controlling the temperature (34) is a Peltier element or a resistive heating element.

8. The transportable pneumotachograph as claimed in one of claims 1 to 7, **characterized in that** the climate control chamber (15) is equipped with a means for setting a defined relative humidity in the climate control chamber (15).

9. The transportable pneumotachograph as claimed in claim 8, **characterized in that** the means for setting the humidity is a water-storing medium, preferably a water-impregnated cotton batting pad, or a hydrophilic medium, preferably made of silica gel.

10. The transportable pneumotachograph as claimed in claim 8, **characterized in that** the means for setting the humidity is a device having two containers (25, 26) each containing a gas with a defined relative humidity, wherein the moisture content of the two gases is different from one another, wherein the containers (25, 26) are connected to the climate control chamber (15) by means of a common regulatable and controllable mixing valve (28), and the relative humidity of the gas in the climate control chamber (15) can be adjusted under process control by means of the mixing valve (28) by mixing the two gases from the containers (25, 26) based on measurements of the moisture sensor (17) which have been converted into control signals.

11. The transportable pneumotachograph as claimed in claim 10, **characterized in that** a heating element (27) is provided for controlling the temperature of the containers (25, 26).

12. The transportable pneumotachograph as claimed in claim 10 or 11, **characterized in that** a mixing chamber (29) with a moisture sensor (31) and a temperature sensor (32) is provided between climate control chamber (15) and mixing valve (28).

13. The transportable pneumotachograph as claimed in one of claims 1 to 12, **characterized in that** the valve (10) is a static three-way valve.

14. The transportable pneumotachograph as claimed in one of claims 1 to 13, **characterized in that** the pneumotachograph tube (2) has a flap (24), which can be closed, in the inlet for inspiration air, wherein the pneumotachograph tube has a plurality of outlets for the expiration air, wherein outlet valves are provided in the outlets for the expiration air, wherein the volume flow of the expiration air can be measured by means of a flow meter and on the basis of its value can be resolved into theoretical values of partial volume flows by means of a processor-assisted calculation, wherein the partial volume flows can be assigned to different zones of the respiration tract, wherein the valve or the flap (24) in the inlet for the inspiration air and/or the outlet valves can be controlled and regulated by the processor (4), wherein the "closed" (a) or "open" (b) operating state of the valve or flap (24) in the inlet for the inspiration air and/or the outlet valves can be set according to a defined partial volume flow of the volume flow of the expiration air.

15. The transportable pneumotachograph as claimed in one of claims 1 to 14, **characterized in that** the PEEP valve (5) is a double valve which limits the flow between a minimum flow and a maximum flow.

16. The transportable pneumotachograph as claimed in one of claims 1 to 15, **characterized in that** the chemically and/or physically modified tube (18) is completely or partially filled with a filter material and/or the inner wall of the tube is coated with a chemically or physically active film in order to remove compounds in the sample or calibration gas which interfere with the sensor measurement.

17. The transportable pneumotachograph as claimed in one of claims 1 to 16, **characterized in that** a processor (14) is provided for controlling and regulating the climate control chamber (15) and/or the mixing chamber (29).

18. The transportable pneumotachograph as claimed in one of claims 1 to 17, **characterized in that** one or more temperature and/or moisture sensors are provided in the pipe carrying sample and/or calibration gas before and/or after the climate control chamber.

## Revendications

1. Pneumotachygraphe transportable pour la détermination de monoxyde d'azote (NO) dans une plage de mesure de 0 à 500 ppb dans le volume d'expiration, comprenant un processeur (4), une vanne en PEEP (5) placée sur le pneumotachygraphe (1) sur le côté expiration, un filtre (6) placé sur le pneumotachygraphe (1) sur le côté inspiration, destiné à éliminer la teneur en NO dans l'air d'inspiration, au moins une prise d'échantillon (8) placée dans ou sur le tube (2) du pneumotachygraphe, une vanne (10) reliée à la prise d'échantillon (8), au moins un capteur (7) agencé à l'extérieur du tube du pneumotachygraphe pour la détermination du NO dans le volume d'expiration et une pompe (13) disposée en aval du capteur (7), **caractérisé en ce qu'**entre la vanne (10) et le capteur (7), est disposé un tuyau (18) modifié chimiquement et/ou physiquement, qui est entouré d'une chambre de climatisation (15) servant à conditionner le gaz échantillon /ou le gaz d'étalonnage qui passe dans le tuyau (18), l'échantillon à conditionner et/ou le gaz d'étalonnage pouvant être conduits dans le tuyau à travers la chambre de climatisation et une atmosphère gazeuse définie pouvant être réglée dans la chambre de climatisation (15), de sorte qu'au moyen de la chambre de climatisation (15), il est possible de régler l'humidité relative et/ou la température et/ou la composition du gaz échantillon/ou d'étalonnage qui circule dans la tuyau (18) sur des paramètres pré-établis.

2. Pneumotachygraphe transportable selon la revendication 1, **caractérisé en ce que** l'humidité relative du gaz qui circule dans le tuyau (18) peut être réglée sur des valeurs de 0 % à 100 % d'humidité relative.

3. Pneumotachygraphe transportable selon l'une des revendications 1 ou 2, **caractérisé en ce que** le tuyau (18) modifié chimiquement et/ou physiquement est perméable à la vapeur d'eau et est, de préférence, un tuyau en Nation®.

4. Pneumotachygraphe transportable selon l'une des revendications 1 à 3, **caractérisé en ce qu'**un capteur d'humidité (17) et/ou un capteur de température (16) sont agencés dans la chambre de climatisation (15).

5. Pneumotachygraphe transportable selon l'une des revendications 1 à 4, **caractérisé en ce que** le débit volumique du gaz dans le tuyau (18) est de 1 à 20 ml/s, de préférence de 2 à 3 ml/s, et peut être réglé en fonction du temps de balayage de la chambre de mesure (14) du capteur (7) et du système d'amenée (8, 9, 10, 18, 33, 35) aboutissant au capteur (7).

6. Pneumotachygraphe transportable selon l'une des revendications 1 à 5, **caractérisé en ce que** la chambre de climatisation (15) est équipée d'un moyen de mise à température (34) de la chambre de climatisation, qui est de préférence réglable et susceptible d'être commandé.

7. Pneumotachygraphe transportable selon la revendication 6, **caractérisé en ce que** le moyen de mise à température (34) est un élément Peltier ou un élément chauffant à résistance.

8. Pneumotachygraphe transportable selon une des revendications 1 à 7, **caractérisé en ce que** la chambre de climatisation (15) est équipée d'un moyen servant à établir une humidité relative définie dans la chambre de climatisation (15).

9. Pneumotachygraphe transportable selon la revendication 8, **caractérisé en ce que** le moyen servant à établir l'humidité est un milieu d'accumulation d'eau, de préférence un tampon d'ouate imprégné d'eau, ou un milieu de rétention d'eau, de préférence fait de gel de silice.

10. Pneumotachygraphe transportable selon la revendication 8, **caractérisé en ce que** le moyen servant à établir l'humidité est un dispositif présentant deux récipients (25, 26) dont chacun contient un gaz ayant une humidité relative définie, les teneurs en humidité des deux gaz étant différentes l'une de l'autre, les récipients (25, 26) étant reliés à la chambre de climatisation (15) au moyen d'un robinet mélangeur commun (28) qui peut être réglé et commandé, et l'humidité relative du gaz contenu dans la chambre de climatisation (15) pouvant être établie par mélange des deux gaz provenant des récipients (25, 26), au moyen du robinet mélangeur (28), en fonction du processus, sur la base de mesures du capteur d'humidité (17) converties en signaux de commande.

11. Pneumotachygraphe transportable selon la revendication 10, **caractérisé en ce qu'**un élément chauffant (27) est agencé pour la mise à température des récipients (25, 26).

12. Pneumotachygraphe transportable selon l'une des revendications 10 ou 11, **caractérisé en ce qu'**entre la chambre de climatisation (15) et le robinet mélangeur (28), est agencée une chambre de mélange (29) équipée d'un capteur d'humidité (31) et d'un capteur de température (32).

13. Pneumotachygraphe transportable selon l'une des revendications 1 à 12, **caractérisé en ce que** la vanne (10) est une vanne statique à trois voies.

14. Pneumotachygraphe transportable selon l'une des revendications 1 à 13, **caractérisé en ce que** le tube (2) du pneumotachygraphe présente, dans l'entrée pour l'air inspiratoire, une vanne ou un clapet (24) qui peut se fermer, le tube du pneumotachygraphe présentant plusieurs sorties pour l'air expiratoire, des vannes de sortie étant agencées dans les sorties pour l'air expiratoire, le débit volumique de l'air expiratoire pouvant être mesuré au moyen d'un débitmètre et pouvant être décomposé en valeurs théoriques de débits volumiques partiels, sur la base de cette valeur, au moyen d'un calcul assisté par processeur, les débits volumiques partiels pouvant être attribués à différentes zones de l'appareil respiratoire, la vanne ou le clapet (24) placé dans l'entrée pour l'air inspiratoire et/ou les vannes de sortie pouvant être pilotés et réglés par le processeur (4), l'état de fonctionnement « fermé » (a) ou « ouvert » (b) de la vanne ou du clapet (24) placé dans l'entrée pour l'air inspiratoire et/ou les vannes de sortie pouvant être commandé en fonction d'un débit volumique partiel déterminé du débit volumique de l'air expiratoire.

15. Pneumotachygraphe transportable selon l'une des revendications 1 à 14, **caractérisé en ce que** la vanne en PEEP (5) est une vanne double qui limite le débit entre un débit minimum et un débit maximum.

16. Pneumotachygraphe transportable selon l'une des revendications 1 à 15, **caractérisé en ce que**, pour l'élimination de composés perturbateurs de la mesure des capteurs qui sont contenus dans le gaz échantillon ou le gaz d'étalonnage, le tuyau (18) modifié chimiquement et/ou physiquement est rempli entièrement ou partiellement d'une matière filtrante et/ou la paroi interne du tuyau est revêtue d'un film chimiquement ou physiquement actif.

17. Pneumotachygraphe transportable selon l'une des revendications 1 à 16, **caractérisé en ce qu'**il comporte un processeur (14) pour la commande et la régulation de la chambre de climatisation (15) et/ou de la chambre de mesure (29).

18. Pneumotachygraphe transportable selon l'une des revendications 1 à 17, **caractérisé en ce qu'**un ou plusieurs capteurs de température et/ou d'humidité sont agencés dans la conduite qui conduit le gaz échantillon et/ou le gaz d'étalonnage en amont et/ou en aval de la chambre de climatisation.
